**Europäisches Patentamt**

**European Patent Office**

**Office européen des brevets**

(19)

(11) Numéro de publication: **0 079 279**
**B1**

(12) **FASCICULE DE BREVET EUROPEEN**

(45) Date de publication du fascicule du brevet:
**22.05.85**

(21) Numéro de dépôt: **82402029.1**

(22) Date de dépôt: **04.11.82**

(51) Int. Cl.⁴: **A 61 J 1/08,** A 61 L 2/26,
C 03 B 23/00

(54) Procédé pour l'ouverture aseptique et antiparticulaire d'enceintes en verre scellé et dispositif et appareil mettant en oeuvre ce procédé.

(30) Priorité: **05.11.81 FR 8120734**

(43) Date de publication de la demande:
**18.05.83 Bulletin 83/20**

(45) Mention de la délivrance du brevet:
**22.05.85 Bulletin 85/21**

(84) Etats contractants désignés:
**BE CH DE GB IT LI NL**

(56) Documents cités:
**DE - A - 2 719 684**
**FR - A - 2 290 496**
**GB - A - 191 581**
**GB - A - 533 473**
**US - A - 2 224 486**
**US - A - 2 704 418**
**US - A - 3 188 191**
**US - A - 3 923 487**

(73) Titulaire: **MILLIPORE S.A., Zone Industrielle,**
**F-67120 Molsheim (FR)**

(72) Inventeur: **Lemonnier, Jean, 63 Allée de la Meute,**
**F-78110 Le Vesinet (FR)**

(74) Mandataire: **Rinuy, Santarelli et al, 14, avenue de la**
**Grande Armée, F-75017 Paris (FR)**

ACTORUM AG

## Description

La présente invention concerne un procédé pour l'ouverture aseptique et antiparticulaire d'enceintes en verre scellé, en vue notamment de permettre un essai de stérilité du contenu de ces enceintes. L'invention concerne également le dispositif et les appareils mettant en œuvre ce procédé.

Comme le recommandent les diverses pharmacopées, tout produit, spécialement tout produit destiné à être injecté dans le corps humain par voie intraveineuse, intramusculaire ou parentérale, doit faire l'objet d'un essai de stérilité pour s'assurer de l'absence de micro-organismes tels que bactéries, champignons ou moisissures, voire même de substances solides étrangères.

Il est évident que, lors du contrôle de nombreux échantillons de produits, par exemple de solutions, de solutés, voire même de produits solides ou en poudre contenus dans des ampoules, il est important de travailler dans des conditions fiables d'asepsie si l'on veut éviter au maximum les risques de faux positifs, cela principalement lors de l'ouverture de telles enceintes ou ampoules et lors du transfert du produit vers le système de filtration auquel on a recours pour effectuer ce contrôle.

On connaît actuellement des systèmes d'ouverture de ces ampoules qui font appel à des moyens mécaniques impliquant soit un limage du col de l'ampoule suivi d'une action de percussion, soit un sciage du col éventuellement suivi d'une action de chauffage et/ou de percussion. Cette façon de procéder présente non seulement le risque que l'ampoule se brise en dehors de la zone voulue et que le produit qu'elle contient se répande inopinément, devenant par conséquent inutilisable pour l'essai de stérilité, mais également le risque d'introduction de particules de verre contaminé dans le milieu à soumettre à cet essai. Ce risque est d'autant plus marqué que ces moyens mécaniques d'ouverture provoquent la formation de poussières ou de fins éclats de verre pouvant facilement atteindre et souiller le produit à analyser.

Par ailleurs, on sait provoquer l'ouverture d'une ampoule par échauffement du verre aux endroits de plus faible résistance du col, sans en atteindre la fusion, cette ouverture se faisant par explosion provoquée par la détente de l'air occlus dans la zone de l'échauffement du verre. Cette façon de procéder présente un inconvénient en ce sens que l'ouverture se produit dans cette zone de plus faible résistance, c'est-à-dire latéralement et aléatoirement et qu'il devient alors malaisé d'y introduire automatiquement l'aiguille de type hypodermique dont on se sert pour effectuer le prélèvement du contenu de l'ampoule.

Vouloir par contre aller jusqu'à la fusion du verre pour réaliser une ouverture suivant l'axe longitudinal de l'ampoule pour rendre possible l'introduction de ladite aiguille hypodermique suivant cet axe présente à son tour l'inconvénient, d'une part, du risque d'un ramollissement du verre à un point tel que l'orifice d'abord réalisé se referme de lui-même par coulage, déformation du verre fondu des lèvres dudit orifice et, d'autre part, du risque d'échauffement inconsidéré du contenu de l'ampoule résultant du chauffage prolongé nécessaire pour la réalisation d'un orifice correct, ce chauffage affectant de proche en proche les parois de l'ampoule et directement ou indirectement son contenu.

Quant à la possibilité de percer une ampoule de verre à l'aide d'un outil pointu porté à très haute température, on conçoit aisément les inconvénients tels que l'éclatement du verre par choc thermique et les risques qui en découlent soit au niveau de la fiabilité, soit au niveau de la mise en œuvre de ce procédé.

Or, la présente invention fournit un procédé qui pallie ces différents inconvénients en ce sens qu'il permet une ouverture aseptique des ampoules de verre de façon contrôlée et reproductible, tout en évitant toute contamination ou échauffement du milieu contenu dans ces ampoules, qu'il peut être appliqué à tout type d'ampoules, qu'il peut être aisément mis en œuvre et utilisé dans un appareil facilement automatisable.

Le procédé de l'invention fait appel à la fusion du verre et il est uniquement caractérisé par le fait que l'on soumet l'une des extrémités de l'ampoule en verre à un flux thermique puissant sur une zone extrêmement localisée pendant une durée suffisante pour y obtenir la fusion du verre, tout en maintenant la fraction plane ou tronconique voisine à une température inférieure à la température de fusion pour lui conserver sa rigidité, mais cette durée étant suffisamment courte pour qu'un faible volume du gaz contenu dans l'ampoule soit amené rapidement à une élévation de température et pression conduisant à l'ouverture de l'ampoule dans la zone fondue considérée.

De façon avantageuse, l'étendue de ladite zone localisée se situe au voisinage de l'axe longitudinal de l'ampoule à ouvrir.

L'invention fournit également un dispositif pour la mise en œuvre de ce procédé, ce dispositif étant lui-même caractérisé par le fait qu'il comporte uniquement un moyen de chauffage dirigé vers une zone localisée d'une des extrémités de l'ampoule et consistant en au moins une source de chaleur génératrice d'un flux thermique intense sous la forme d'un dard de très haute température voisine de la température de fusion du verre, ce dard étant dirigé de manière à se trouver dans un plan sensiblement tangent à la surface du verre et au voisinage de l'axe longitudinal de l'ampoule.

Suivant un mode de réalisation avantageux, ce dispositif peut comporter deux sources génératrices de dard se faisant face et disposées de manière à se rencontrer pour former un halo en forme de disque sensiblement vertical dont une partie de la périphérie est tangente à la surface du verre à traiter.

Suivant une variante, le dispositif comporte plus de deux sources disposées pour former un halo dont le centre est situé sur l'axe longitudinal de l'ampoule.

Suivant d'autres caractéristiques, la ou les sources génératrices de dard sont montées sur une fourche susceptible d'être animée d'un mouvement ascendant-descendant de manière à amener le ou les dards et/ou le halo dans la position relative voulue par rapport à la surface du verre à traiter.

Le procédé et le dispositif selon la présente invention peuvent aisément être utilisés dans un appareil pour essai de stérilité de produits contenus dans des ampoules scellées, appareil comprenant alors:

— un poste d'ouverture des ampoules par fusion du verre comportant le dispositif tel que défini ci-dessus;

— un moyen de présentation audit poste d'ouverture des ampoules constituant les échantillons de produit à tester;

— un poste de prélèvement, relié à un appareil d'essai de stérilité.

Un tel appareil peut être rendu à fonctionnement automatique, c'est-à-dire que les ampoules à ouvrir seront successivement ouvertes, leur contenu prélevé puis admis dans le système d'analyse proprement dit.

Pour ce faire, le moyen de présentation des ampoules au poste d'ouverture peut être constitué par un plateau porte-échantillon animé d'un mouvement d'avancement pas à pas ne s'effectuant qu'après l'ouverture d'une ampoule, et le poste de prélèvement peut être constitué par un dispositif porte-aiguille animé d'un mouvement ascendant-descendant agissant en synchronisme avec, d'une part, le mouvement ascendant-descendant du dispositif du poste d'ouverture et, d'autre part, le mouvement pas à pas du plateau porte-échantillon, ce poste de prélèvement étant lui-même relié à l'appareil d'essai de stérilité dont le dispositif d'aspiration est lui-même actionné en synchronisme avec les mouvements ascendant-descendant du dispositif porte-aiguille.

L'appareil mettant en œuvre le procédé et le dispositif d'ouverture selon l'invention comprend alors un boîtier de commande et les organes qui y sont associés, boîtier et organes étant de type connu pour la synchronisation des mouvements de chacun des moyens constitutifs de cet appareil.

Suivant une caractéristique, l'appareil peut comporter, pour ce qui est du poste de prélèvement d'échantillon associé au dispositif porte-aiguille, un dispositif de vérification de la stérilité tel que celui décrit dans le brevet français No 75.33838 (publié sous le No 2290496).

D'autres caractéristiques et les avantages de l'invention ressortiront plus clairement de la description qui va suivre faite en regard des dessins annexés sur lesquels:

— les fig. 1a et 1b sont des schémas illustrant le procédé selon l'invention;

— les fig. 1c et 1d sont des vues partielles correspondantes suivant les flèches f-f;

— les fig. 2a et 2b illustrent des ampoules ouvertes selon l'invention;

— la fig. 3 est une vue en élévation avec coupe partielle d'un dispositif mettant en œuvre le procédé de l'invention;

— la fig. 4 est une vue en perspective d'un exemple d'un ensemble d'un appareil automatique mettant en œuvre le procédé et le dispositif d'ouverture selon l'invention appliqués à l'essai de stérilité d'ampoules de solutés, et

— les fig. 5a et 5b illustrent les avantages que l'on peut obtenir en mettant en œuvre le procédé de l'invention dans le cas d'ampoules de différentes capacités.

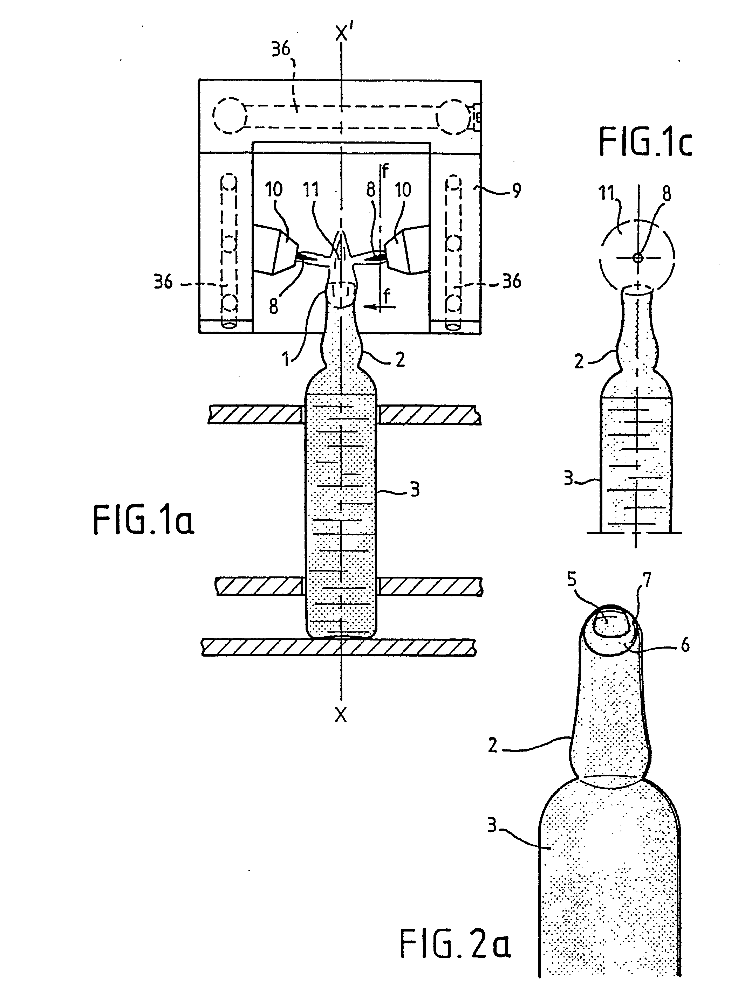

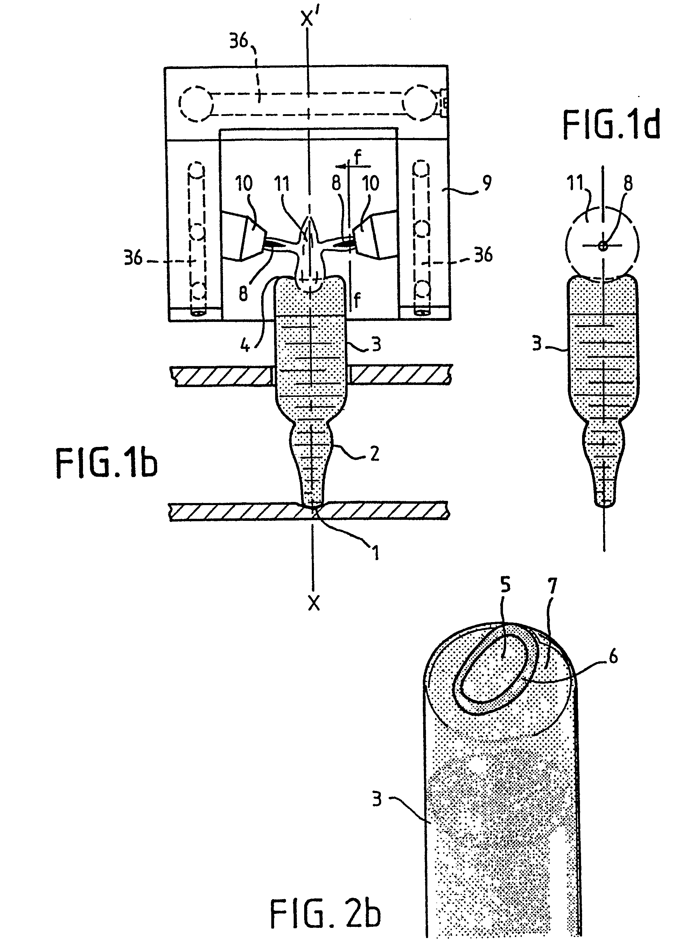

En se référant à ces figures, le procédé de l'invention consiste à amener à l'état fondu une zone extrêmement limitée d'une ampoule de verre, à l'exclusion des zones environnantes qui, elles, ne sont portées qu'à une température inférieure à la température de fusion du verre. Dans le cas pris comme exemple et illustré par les fig. 1a et 1b, c'est l'extrémité supérieure ou pôle de la calotte 1 terminant le col 2 de l'ampoule 3 ou sa base inférieure 4 qui est portée à la température de fusion du verre.

Sur cette zone ainsi rendue nettement plus fragile, s'exerce alors la pression du volume d'air ayant été de ce fait chauffé, pression qui se traduit par la percée du verre à cet endroit par l'évacuation brutale de cet air sans déformation des zones environnantes. En effet, en l'absence de choc thermique sur ces zones environnantes qui sont elles-mêmes portées à température élevée sans atteindre toutefois la température de fusion, ces zones, d'une part, n'ont pas tendance à se briser de façon incontrôlée et, d'autre part, n'ont pas tendance à fluer pour obturer l'orifice ainsi réalisé du fait qu'elles ne sont pas amenées à l'état fondu permettant un tel fluage.

D'où l'intérêt, pour une bonne réalisation du procédé, d'effectuer la fusion du verre sur une surface restreinte pendant une durée le plus court possible pour éviter que de proche en proche le verre ne soit amené à l'état fondu sur une trop grande surface, d'où une déformation du verre, et partant la déformation des lèvres de l'orifice réalisé, pouvant aller jusqu'à son obturation. On comprendra alors la nécessité qu'il y a de soumettre la zone dont on veut provoquer la fusion rapidement à une température élevée, au moins égale à la température de fusion du verre de manière à amener rapidement un petit volume d'air occlus à une température telle que la pression en résultant s'exerce aussi rapidement que possible sur la zone de verre rendue fragile par suite de la fusion. Les fig. 2a-2b montrent le résultat obtenu grâce à ce procédé. On voit en effet sur ces figures les orifices 5 réalisés à l'extrémité des ampoules 3. Le bourrelet 6 en constitue les lèvres. Il résulte de l'échappement brutal des gaz chauds occlus, qui n'a eu pour effet que de rabattre et d'écarter le verre fondu sur et vers la zone environnante 7 demeurée intacte et n'ayant subi aucun choc thermique.

Pour obtenir ce résultat, le dispositif selon l'invention et qui met en œuvre ce procédé comprend au moins une source génératrice d'une flamme affectant la forme d'un dard très étroit et dirigé de façon à raser la zone dans laquelle l'orifice doit être réalisé.

Sur les fig. 1a-1b, on a représenté un ensemble en forme d'étrier 9 portant sur ses deux branches opposées deux ajutages ou injecteurs 10 reliés chacun à une source non représentée d'un gaz ou d'un mélange de gaz combustible, le diamètre de ces ajutages étant réglé de manière à réaliser les deux dards 8 dont les extrémités se rejoignent pour former un halo 11 en forme de disque sensiblement vertical et tangent à la surface du verre à percer.

Comme on peut le constater à l'examen de ces figures, le halo où règne la température la plus élevée susceptible de conduire à la fusion du verre n'affecte que le pôle de la calotte du col de l'ampoule ou sa base sur une zone restreinte, les zones environnantes n'étant soumises qu'à une température plus basse et par conséquent échappant au phénomène de fusion.

Suivant l'inclinaison que l'on donne au halo en faisant varier l'inclinaison des dards ou le débit de gaz dans l'un ou l'autre des injecteurs, l'orifice peut être réalisé en un point favorable au voisinage ou le plus près possible de l'axe longitudinal X-X' de l'ampoule pour faciliter au mieux les opérations de prélèvement de son contenu.

Dans le cas où l'orifice doit être réalisé sur le fond 4 d'une ampoule, le résultat obtenu selon l'invention est illustré sur la fig. 2b.

L'analyse de ce résultat conduit immédiatement aux remarques suivantes:

a) Le procédé permet de pratiquer de façon contrôlée, de façon reproductible et de façon aseptique, étant donné l'absence de toute cause de pollution ou d'apport de corps étrangers, des ouvertures dans des ampoules de verre au voisinage immédiat de leur axe longitudinal.

b) L'air occlus dans ces ampoules n'est chauffé que sur un volume relativement petit, ce qui met à l'abri le contenu de ces ampoules contre tout échauffement inconsidéré et par conséquent contre toute altération pouvant être provoquée par des variations thermiques.

c) Etant donné la localisation de l'ouverture pratiquée, une aiguille de prélèvement du contenu d'une ampoule peut y être introduite aisément et verticalement.

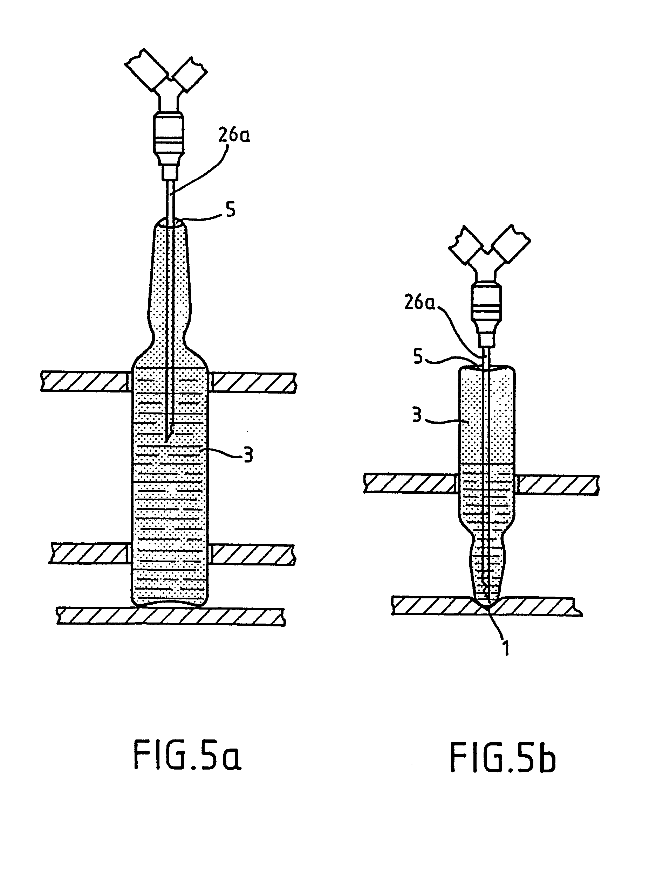

d) La possibilité de pratiquer un orifice axial aussi bien dans le col que dans le fond d'une ampoule permet d'effectuer des prélèvements sur des ampoules aussi bien en position debout (voir fig. 5a — cas d'une ampoule de plus de 1 ml sur laquelle il est possible de prélever le volume de soluté voulu) qu'en position renversée (voir fig. 5b — cas d'une ampoule de moins de 1 ml de laquelle la totalité du soluté doit être prélevée).

Etant donné les avantages et l'intérêt du procédé et du dispositif selon l'invention, ceux-ci peuvent être mis à profit dans la réalisation d'un appareil pouvant être utilisé dans un ensemble automatique destiné au prélèvement aseptique de contenus d'ampoule en vue de l'essai de stérilité.

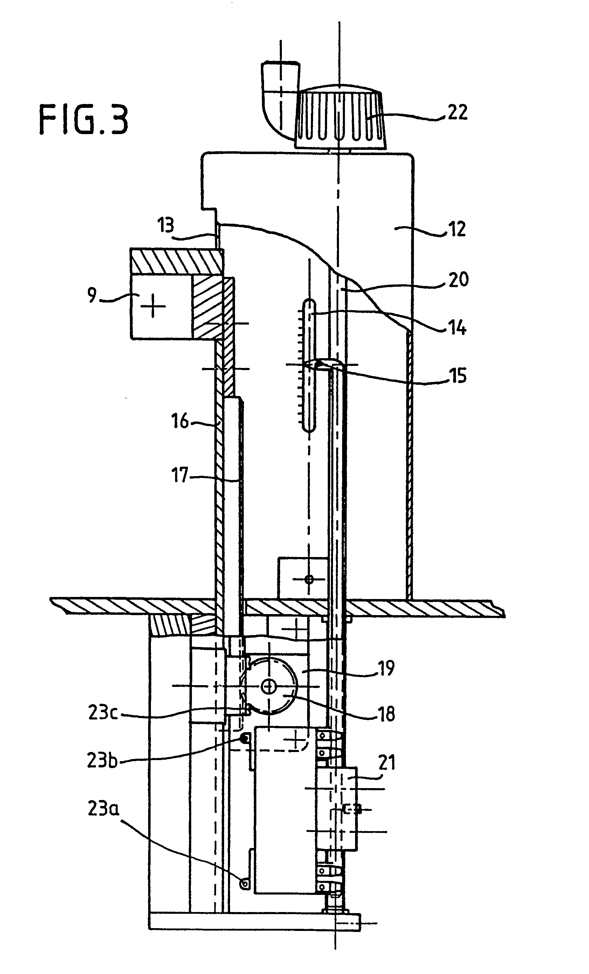

Un tel appareil est représenté sur la fig. 3. Il est essentiellement constitué par une pièce cylindrique 12 comportant une fenêtre 13 dans laquelle est susceptible de coulisser, comme on le verra ci-après, l'étrier 9 porteur des injecteurs 10 dont il a été question ci-dessus. Par ailleurs, le long d'une génératrice de cette pièce cylindrique, est réalisée une fente longitudinale 14 portant une échelle de graduations devant lesquelles peut se déplacer un curseur 15 de la façon qui sera décrite ci-après et dont le rôle ressortira également de cette description.

L'étrier 9 est porté par une plaque 16 rendue solidaire d'une crémaillère 17 en prise avec un engrenage 18 susceptible d'être entraîné en rotation par tout moyen approprié, par exemple un moteur électrique 19.

L'index ou curseur 15 est monté, par l'intermédiaire d'un écrou 21, sur un arbre 20 dont la rotation peut être commandée par un bouton-manivelle 22 pour amener ledit curseur à la position voulue en regard de la graduation choisie de l'échelle graduée de la fente 14. Cette position règle l'amplitude du mouvement ascendant-descendant de l'étrier 9, par exemple par une liaison électrique s'établissant par des contacts 23a, 23b, 23c prévus sur la partie inférieure de la pièce porte-crémaillère et sur la pièce porte-index de manière à commander électriquement le moteur 19, et partant la rotation de l'engrenage 18 en prise avec la crémaillère 17.

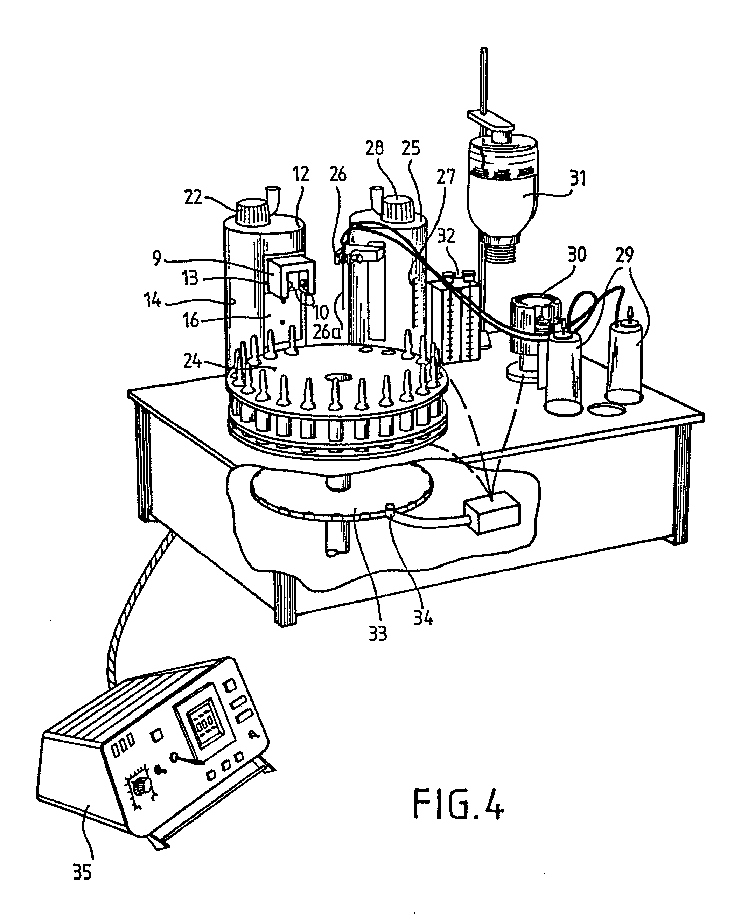

Un tel appareil peut être utilisé comme indiqué ci-dessus dans un ensemble entièrement automatique illustré à la fig. 4, permettant d'effectuer un essai de stérilité.

Cet ensemble comporte essentiellement le poste d'ouverture des ampoules 12 tel qu'il vient d'être décrit, coopérant avec un dispositif de présentation des ampoules à ouvrir. Ce dernier peut être constitué par exemple par un plateau porte-ampoules 24 susceptible d'être animé d'un mouvement de rotation pas à pas, comme on le verra ci-après. La hauteur des ampoules à ouvrir dicte la hauteur à laquelle doit se situer l'étrier porte-générateur du flux thermique de manière à mettre en œuvre le procédé de l'invention tel que précédemment défini.

En association avec ledit poste d'ouverture, l'ensemble comporte un poste de prélèvement du contenu de chaque ampoule, ce poste étant constitué, d'une part, par un élément cylindrique 25 de construction et de fonctionnement identiques à ceux de l'élément 12, à l'exception du fait que l'étrier porte-générateur de flux thermique est remplacé par un porte-aiguille hypodermique 26-26a susceptible d'être animé d'un mouvement ascendant-descendant dont l'amplitude est réglée, comme précédemment, par un curseur amené devant une échelle graduée 27 par le bouton-manivelle 28 suivant la profondeur à laquelle l'aiguille doit pénétrer à l'intérieur d'une ampoule ouverte. L'aiguille hypodermique 26a est directement branchée sur l'appareil de vérification de stérilité. Celui-ci peut être de tout type connu. Il sera avantageusement du type de celui décrit dans le brevet français N° 75.33838 (publié sous le N° 2290496), c'est-à-dire comportant ses deux boîtes spéciales 29 qui ne seront pas décrites ici et dont l'une a été représentée en liaison directe avec l'aiguille hypodermique, d'une part, et la pompe d'aspiration 30, d'autre part. On a également représenté en 31 le réservoir du liquide approprié servant àl'essai de stérilité qu'il n'est pas nécessaire de décrire à nouveau ici.

Sur la fig. 4, on a également fait figurer deux débitmètres 32 réglant le débit du gaz ou du mélange de gaz combustible admis dans le poste d'ouverture des ampoules.

L'automaticité de l'ensemble, dont les détails de réalisation sont à la portée de l'homme de l'art et ne seront pas décrits ici, est obtenue de la façon suivante:

Le plateau 24 est mis en rotation pas à pas grâce à un disque 33 monté sur le même arbre, commandé par un moteur (non représenté) et comportant à sa périphérie un crantage du type roue à rochet en prise avec un cliquet contacteur 34 commandant la descente ou la montée de l'étrier 9, la montée ou la descente du porte-aiguille 26 et la mise en route de la pompe d'aspiration 30.

Un boîtier 35 est prévu pour la commande et la surveillance générale de la bonne marche de l'ensemble.

Ainsi, un cycle d'essai de stérilité d'un échantillon de produit contenu dans une ampoule comporte les stades suivants:

a) présentation d'une ampoule au poste d'ouverture; arrêt du plateau 24 lorsqu'une ampoule scellée arrive au droit de l'étrier 9;

b) descente de l'étrier à la hauteur voulue au-dessus de l'ampoule et action du flux thermique suivant le procédé de l'invention: ouverture de l'ampoule;

c) avance du disque jusqu'à ce que l'ampoule ainsi ouverte se trouve au droit de l'aiguille hypodermique du poste de prélèvement 25; pendant le même temps, l'étrier 9 est remonté pour être prêt à l'opération suivante;

d) arrêt du disque 24, descente de l'aiguille hypodermique à l'intérieur de l'ampoule ouverte, action d'aspiration de la pompe 30 et admission du produit en 29; pendant le même temps, ouverture de l'ampoule suivante, et le cycle se poursuit.

Les dispositifs et circuits électriques de commande de ce cycle sont de type classique et peuvent être réalisés de toute façon appropriée connue en soi.

Pour éviter un échauffement exagéré de l'étrier du poste d'ouverture, l'étrier 9 peut comporter un circuit de refroidissement constitué par une circulation de fluide à l'intérieur de ses branches. On n'a pas représenté le circuit d'admission du gaz ou du mélange de gaz combustible,d ans un souci de clarté et de simplification.

Bien entendu, l'invention peut être appliquée à l'essai de stérilité de produits secs (poudres solubles ou lyophilisées). Dans ce cas, un poste de solubilisation du produit est prévu entre le poste d'ouverture et le poste de prélèvement.

**Revendications**

1. Procédé pour l'ouverture aseptique et antiparticulaire d'une ampoule (3) en verre scellé contenant un produit liquide ou solide par fusion du verre tout en protégeant son contenu contre toute élévation de température, procédé uniquement caractérisé par le fait que l'on soumet l'une des extrémités (1, 4) de l'ampoule (3) en verre à un flux thermique puissant sur une zone extrêmement localisée (5) pendant une durée suffisante pour y obtenir la fusion du verre tout en maintenant la fraction plane ou tronconique voisine (7) à une température inférieure à la température de fusion pour lui conserver sa rigidité, mais cette durée étant suffisamment courte pour qu'un faible volume du gaz contenu dans l'ampoule soit amené rapidement à une élévation de température et à une pression conduisant à l'ouverture de l'ampoule dans la zone fondue (5) considérée.

2. Procédé selon la revendication 1, caractérisé par le fait que l'étendue de ladite zone localisée (5) se situe au voisinage de l'axe longitudinal (X-X') de l'ampoule (3) à ouvrir.

3. Dispositif d'ouverture pour la mise en œuvre du procédé selon l'une des revendications 1 ou 2, caractérisé par le fait qu'il comporte uniquement un moyen de chauffage dirigé vers une zone localisée (5) d'une des extrémités (1, 4) de l'ampoule (3) et consistant en au moins une source de chaleur (10) génératrice d'un flux thermique intense sous la forme d'un dard (8) de très haute température, voisine de la température de fusion du verre, ce dard (8) étant dirigé de manière à se trouver dans un plan sensiblement tangent à la surface du verre et au voisinage de l'axe longitudinal (X-X') de l'ampoule (3).

4. Dispositif selon la revendication 3, caractérisé par le fait qu'il peut comporter deux sources génératrices (10) de dard se faisant face et disposées de manière à se rencontrer pour former un halo (11) en forme de disque sensiblement vertical dont une partie de la périphérie est tangente à la surface de verre à traiter.

5. Dispositif selon la revendication 3, caractérisé par le fait qu'il peut comporter plus de deux sources disposées pour former un halo dont le centre est situé sur l'axe longitudinal (X-X') de l'ampoule (3).

6. Dispositif selon l'une des revendications 3 à 5, caractérisé par le fait que la ou les sources génératrices (10) de dard sont montées sur une fourche (9) susceptible d'être animée d'un mouvement ascendant-descendant de manière à amener le ou les dards (8) et/ou le halo (11) dans la position relative voulue par rapport à la surface du verre à traiter.

7. Application du dispositif selon l'une quelconque des revendications 3 à 6 dans un appareil pour essai de stérilité de produits contenus dans des ampoules scellées (3), appareil caractérisé par le fait qu'il comprend:
— ledit dispositif d'ouverture (12) des ampoules par fusion du verre;
— un moyen de présentation (24) audit dispositif d'ampoules constituant les échantillons de produits à tester, et
— un poste de prélèvement (25) du contenu de l'ampoule présentée et ouverte relié à un appareil d'essai de stérilité (29) de ce contenu.

8. Application selon la revendication 7 dans un appareil rendu à fonctionnement automatique dans lequel le moyen de présentation des ampoules au poste d'ouverture (12) peut être constitué par un plateau porte-échantillon (24) animé d'un mouvement d'avancement pas à pas ne s'effectuant qu'après l'ouverture d'une ampoule, et le poste de prélèvement peut être constitué par un dispositif porte-aiguille (25) animé d'un mouvement ascendant-descendant agissant en synchronisme avec, d'une part, le mouvement ascendant-descendant du dispositif du poste d'ouverture et, d'autre part, le mouvement pas à pas du plateau porte-échantillon (24), ce poste de prélèvement étant lui-même relié à l'appareil d'essai de stérilité (29) dont le dispositif d'aspiration (30) est lui-même actionné en synchronisme avec les mouvements ascendant-descendant du dispositif porte-aiguille (25).

9. Application selon la revendication 8, dans laquelle ledit appareil à fonctionnement automatique comprend alors un boîtier de commande (35) et les organes qui y sont associés, boîtier et organes étant de type connu pour la synchronisation des mouvements de chacun des moyens constitutifs de cet appareil.

**Claims**

1. A method of aseptic and antiparticulate opening of a sealed glass ampul (3) containing a liquid or solid product by melting glass thereof while protecting its content from any temperature increase, this method being only characterized in that one of the ends (1, 4) of the glass ampul (3) is submitted to a powerful thermal flux over a very localized zone (5) for a time period sufficient to obtain the melting of the glass while holding the adjacent plane or frustoconical fraction (7) at a temperature lower than the melting temperature to preserve rigidity thereof, such time period being however sufficiently short so that a little volume of gas contained in the ampul is quickly brought to an increased temperature and pressure leading to the opening of the ampul in the considerer molten zone (5).

2. A method according to Claim 1, characterized in that the extent of said localized zone (5). lies in the neighbourhood of the longitudinal axis X-X' of the ampul (3) to be opened.

3. An opening device for carrying out the method according to any of Claims 1 or 2, characterized in that it only comprises heating means directed to a localized zone (5) of one of the ends (1, 4) of the ampul (3) and consisting of at least a heat source (10) productive of an intense thermal flux in form of a very high temperature flame tongue (8) close to the temperature of glass melting, such tongue (8) being directed so as to lie in a plane substantially tangent to the glass surface and in the neighbourhood of the longitudinal axis (X-X') of the ampul (3).

4. A device according to Claim 3, characterized in that it may comprise two flame tongue producing sources (10) facing each other and disposed so as to meet and form a substantially vertical disk-shaped halo (11), a portion of the periphery of which is tangent to the glass surface to be treated.

5. A device according to Claim 3, characterized in that it may comprise more than two sources disposed so as to form a halo having its center located on the longitudinal axis (X-X') of the ampul (3).

6. A device according to any one of Claims 3 to 5, characterized in that the tongue producing source(s) (10) are mounted to a stirrup (9) capable of being driven into an upward and downward motion so as to bring the tongue(s) (8) and/or the halo (11) to the desired relative position with respect to the glass surface to be treated.

7. The device according to any one of Claims 3 to 6, adapted into an apparatus for testing sterility of products contained in sealed ampuls (3), said apparatus being characterized in that it comprises:
— said device (12) for opening the ampul by melting the glass,
— means (24) for presenting ampul, forming the samples of products to be tested, to said device, and
— a station (25) for extracting the content of the ampul presented to and opened, said station being connected to an apparatus (29) for testing sterility of such content.

8. Apparatus according to Claim 7, adapted to operate automatically, wherein the means for presenting the ampul to the opening station (12) may consist of a sample carrier plate (24) driven into a stepwise forward motion which occurs only after opening an ampul, and the extracting station may consist of a needle carrying device (25) driven into an upward and downward motion of the device of the opening station on the one hand, and, on the other hand, with the stepwise motion of the sample carrier plate (24), the extracting station being itself connected to the sterility test apparatus (29), the suction device (30) of which is itself actuated synchronously with the upward and downward motions of the needle carrying device (25).

9. Apparatus according to Claim 8, wherein said automatic operation apparatus then comprises a control box (35) with the elements associated therewith, the control box and elements being of a known type for synchronization of the motions of each of the constituent means of said apparatus.

**Patentansprüche**

1. Verfahren zum aseptischen und partikelfreien Öffnen von verschlossenen Ampullen aus Glas (3), die ein flüssiges oder festes Produkt enthalten, durch Schmelzen des Glases, wobei ihr Inhalt gegen jegliche Temperaturerhöhung geschützt wird, einzig dadurch gekennzeichnet, dass eines der Enden (1, 4) der Ampulle aus Glas (3) einem starken Wärmestrom auf eine äusserst lokalisierte Zone (5) während einer Dauer unterworfen ist, die ausreicht, um dort das Schmelzen des Glases zu erreichen, während der ebene oder kegelstumpfförmige Nachbarbereich (7) auf einer Temperatur unterhalb der Schmelztemperatur gehalten wird, um seine Steifigkeit zu erhalten, aber diese Dauer ausreichend kurz ist, um ein geringes Volumen des inder Ampulle enthaltenen Gases schnell zu einer Erhöhung von Temperatur und Druck zu bringen, die zur Öffnung der Ampulle in der betrachteten geschmolzenen Zone (5) führt.

2. Verfahren nach Anspruch 1, dadurch gekennzeichnet, dass sich die Ausdehnung der lokalisierten Zone (5) in der Nachbarschaft der Längsachse (X-X') der zu öffnenden Ampulle (3) befindet.

3. Vorrichtung zum Öffnen unter Verwendung des Verfahrens nach einem der Ansprüche 1 oder 2, dadurch gekennzeichnet, dass sie einzig eine auf die lokalisierte Zone (5) eines der Enden (1, 4) der Ampulle (3) gerichtete Heizvorrichtung besitzt und dass sie mindestens eine Wärmequelle (10) zur Erzeugung eines intensiven Wärmestromes in der Form einer Zunge (8) sehr hoher Temperatur in der Nähe der Schmelztemperatur des Glases besitzt, wobei diese Zunge (8) in einer Weise ausgerichtet ist, dass sie sich in einer Ebene im wesentlichen tangential zur Oberfläche des Glases und in der Nachbarschaft der Längsachse (X-X') der Ampulle (3) befindet.

4. Vorrichtung nach Anspruch 3, dadurch gekennzeichnet, dass sie zwei Entstehungsquellen (10) für Zungen aufweisen kann, die sich gegenüberliegen und in einer Weise angeordnet sind, dass sie sich treffen, um einen Hof (11) in der Form einer im wesentlichen senkrechten Scheibe zu bilden, von der ein Teil des Randes die Oberfläche des zu behandelnden Glases berührt.

5. Vorrichtung nach Anspruch 3, dadurch gekennzeichnet, dass sie mehr als zwei Quellen aufweisen kann, die so angeordnet sind, dass sie einen Hof bilden, dessen Zentrum auf der Längsachse (X-X') der Ampulle (3) liegt.

6. Vorrichtung nach einem der Ansprüche 3 bis 5, dadurch gekennzeichnet, dass die Entstehungsquelle(n) (10) von Zungen auf einer Gabel (9) angebracht ist (sind), die geeignet ist, durch eine Aufwärts-Abwärts-Bewegung derart bewegt zu werden, dass die Zunge(n) (8) und/oder der Hof (11) in die in bezug auf die Oberfläche des zu behandelnden Glases gewünschte relative Position geführt werden.

7. Anwendung der Vorrichtung nach irgendeinem der Ansprüche 3 bis 6 in einer Vorrichtung zur Prüfung der Sterilität der in den verschlossenen Ampullen (3) enthaltenen Produkte, wobei die Vorrichtung dadurch gekennzeichnet ist, dass sie enthält:
— die Öffnungsvorrichtung (12) der Ampullen durch Schmelzen von Glas;
— ein Mittel zur Zuführung (24) von Ampullen, die die Proben der zu testenden Produkte darstellen, zur Vorrichtung, und
— eine Entnahmestelle (25) für den Inhalt der herangeführten und geöffneten Ampulle, angeschlossen an ein Gerät zur Überprüfung der Sterilität (29) dieses Inhaltes.

8. Anwendung nach Anspruch 7 in einer Vorrichtung, die dazu geschaffen ist, automatisch zu arbeiten, in der das Mittel zur Zuführung der Ampullen zur Öffnungsstelle (12) als eine Probenträgerplatte (24) eingerichtet sein kann, die durch eine schrittweise Vorwärtsbewegung bewegt ist, die nur nach der Öffnung einer Ampulle zustande kommt, und die Entnahmestelle durch eine Nadelträgervorrichtung (25) dargestellt sein kann, die durch eine Aufwärts-Abwärts-Bewegung bewegt werden kann, die einerseits synchron mit der Aufwärts-Abwärts-Bewegung der Vorrichtung der Öffnungsstelle und andererseits der schrittweisen Bewegung der Probenträgerplatte (24) arbeitet, wobei die Entnahmestelle selbst mit dem Sterilitätsprüfgerät (29) verbunden ist, dessen Saugvorrichtung (30) ihrerseits synchron mit den Aufwärts-Abwärts-Bewegungen der Nadelträgervorrichtung (25) arbeitet.

9. Anwendung nach Anspruch 8, in der die automatisch arbeitende Vorrichtung jetzt ein Steuerteil (35) enthält und die ihm zugeordneten Organe, wobei Steuerteil und Organe von der Art sind, die für die Synchronisation der Bewegungen jeder dieser wesentlichen Einrichtungen dieser Vorrichtung bekannt sind.

FIG.1a

FIG.1c

FIG.2a

FIG.1b

FIG.1d

FIG. 2b

FIG.3

FIG.4

FIG.5a

FIG.5b